# EUROPEAN PATENT SPECIFICATION

(11) **EP 1 707 172 B1**
(45) Date of publication and mention of the grant of the patent: **27.08.2008**
(21) Application number: 06251759.4
(22) Date of filing: 30.03.2006
(51) Int. Cl.: A61F 13/82, A61F 13/84

(54) **Body attachable sanitary protection article including tactile placement aid**
Am Körper klebender Hygieneartikel mit fühlbarer Platzierungshilfe
Article absorbant comprenant un adhesif au corps et une aide tactile de positionnement

(30) Priority: 31.03.2005 US 96086
(43) Date of publication of application: 04.10.2006
(73) Proprietor: McNeil-PPC, Inc., Skillman, NJ 08558 (US)
(72) Inventor: Gannon, Elaine M., Hoboken, NJ 07030 (US); Joseph, Annemarie Devine, Evanston, IL 60201 (US); Moscherosch, H. Michael, Doylestown, PA 19801 (US)
(74) Representative: Mercer, Christopher Paul

(56) References cited:
- EP-A- 0 888 762
- EP-A- 1 407 737
- US-A- 6 045 544

## Description

### FIELD OF THE INVENTION

The present invention relates to a body attachable sanitary protection article, such as a sanitary napkin, and in particular a body attachable sanitary protection article including a tactile placement aid.

### BACKGROUND OF THE INVENTION

Various absorbent articles configured to be arranged adjacent the body to absorb body fluids such as menses, urine and the like are well known. With respect to feminine hygiene, napkins and liners have been developed for external use about the pudendal region of a female.

Securement of a sanitary napkin or liner during use is normally accomplished by attaching the sanitary garment by pressure sensitive adhesive to the wearer's undergarment. Napkins having wings or flaps that fold over the edges of the garment and are attached to the underside of the garment using an adhesive are also known.

The prior art also teaches sanitary protection articles that are intended to be secured directly to the body by an adhesive arranged on a body-facing surface of the article. For example, GB2284767A purports to disclose an absorbent article including an absorbent and an adhesive arranged adjacent the absorbent, the adhesive being designed to contact the wearer's body during use. U.S. Patent No. 6,213,993 purports to disclose a self-adhering absorbent article including a liquid permeable cover, an absorbent core, a liquid impermeable baffle, and a bodyside adhesive arranged on the cover for securing the article to the body.

A problem with body-attachable sanitary articles of the type described above is that it is difficult for the user to arrange the article in the proper location against the body. In particular, user's often have difficulty aligning the product over the vaginal opening since they must place the article against the body on a substantially non-visual basis due to the anatomical location where the article must be adhered to the body. This problem often results in the article being adhered to the body at an improper location which results in a reduction in overall performance of the product. Further, the improper placement of the article may result in failure of the product during use thereby causing fluid leakage.

A shaped absorbent interlabial device, disclosed in US6045544, may have a stiffened central region for improved comfort. The stiffened region may provide a visual indication of how the product should be held by a user during application.

A further interlabial absorbent device, disclosed in EP1407737, may be provided on a reverse surface thereof, with a pocket for locating a finger prior to application of the device by a user.

An alternative absorbent napkin type article, disclosed in EP0888762, may be provided with fold lines giving it a tridimensionality in conformity with the body shape of a user and providing guidance to control the placement of the napkin on the anatomy.

In view of the above there is a need for an improved body attachable sanitary article that overcomes the drawbacks and shortcomings of the articles disclosed in the prior art.

### SUMMARY OF THE INVENTION

According to the invention, there is provided a body-attachable absorbent article comprising a body facing surface; a garment facing surface; an adhesive arranged on said body facing surface for securing said article to the body; and a tactile placement aid perceptible to a user on a non-visual basis and that is tactilely perceptible to a user from said garment facing surface of said article, said tactile placement aid comprising a first tactilely perceptible element and a least a second tactilely perceptible element arranged within said first tactilely perceptible element.

The article of the invention is defined in appended claim 1. Further preferred features thereof are defined in subclaims 2-19.

In a further aspect, the present invention relates to a method of applying the body-attachable absorbent article of the invention to the body, comprising: tactilely locating the tactile placement aid on a body-attachable absorbent article; tactilely aligning the tactile placement aid with a vaginal opening of a user; and adhering the body-attachable absorbent article against a body of the user so that said tactile placement aid is aligned with said vaginal opening of said user.

According to a fourth aspect of the invention, the present invention relates to a method of applying a body-attachable absorbent article to the body, including the steps of:
tactilely locating a tactile placement aid on a body-attachable absorbent article;
tactilely aligning the tactile placement aid with a vaginal opening of a user; and
adhering the body-attachable absorbent article against a body of the user so that said tactile placement aid is aligned with said vaginal opening of said user.

### BRIEF DESCRIPTION OF THE DRAWINGS

Examples of embodiments of the present invention will now be described with reference to the drawings, in which:
Fig. 1 is a perspective view from a garment facing side of body attachable sanitary napkin according to a first embodiment of the present invention with the release member thereof partially removed;
Fig. 2 is a perspective view from a body-facing side of the body attachable sanitary napkin shown in Fig. 1 with the release member thereof partially removed;
Fig.3 is an sectional view of the sanitary napkin shown in Fig. 1 taken along line 3-3 in Fig. 1;
Fig. 4 is an exploded perspective view of the body attachable sanitary napkin shown in Fig. 1;
Fig. 5 is a perspective view from a body facing side of a body attachable sanitary napkin according to a second embodiment of the present invention with the release member thereof partially removed;
Fig. 6 is a perspective view of the sanitary napkin shown in Fig. 1 illustrating a user tactilely locating the placement aid of the sanitary napkin;
Fig. 7 is a perspective view from a garment facing side of a body attachable sanitary napkin according to a third embodiment of the present invention;
Fig. 8 is a detailed view of the sanitary napkin in Fig. 7 showing a portion of the tactile placement aid thereof;
Fig. 9 is a perspective view from a garment facing side of a body attachable sanitary napkin according to a fourth embodiment of the present invention with the release member thereof partially removed; and
Fig. 10 is an exploded perspective view of the sanitary napkin in Fig. 9.

### DETAILED DESCRIPTION OF THE INVENTION

Specific embodiments of the present invention will be described in detail below with reference to the cited figures wherein similar or identical components have been identified with the same or similar identifying numbers. The present disclosure refers to a few specific embodiments of the invention however other embodiments within the scope of the invention will be apparent to those skilled in the art.

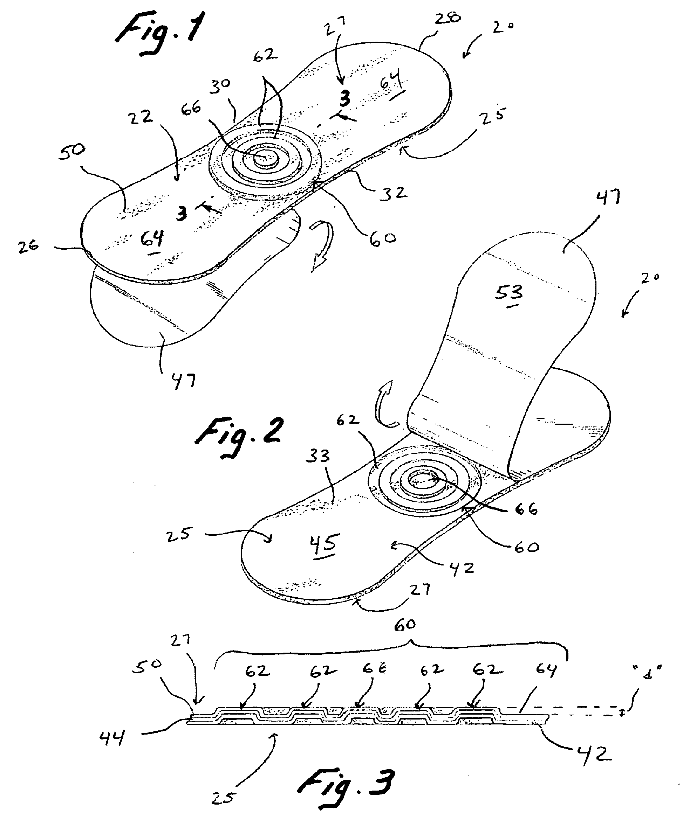

Referring to FIGS. 1-4, there is shown a first embodiment of the present invention, a feminine sanitary napkin 20. The sanitary napkin 20 has a main body 22 with a first transverse side 26 defining a front portion thereof and a second transverse side 28 defining a rear portion thereof. The main body also has two longitudinal sides, namely a longitudinal side 30 and a longitudinal side 32. The main body further includes a body facing surface 25 and a garment facing surface 27.

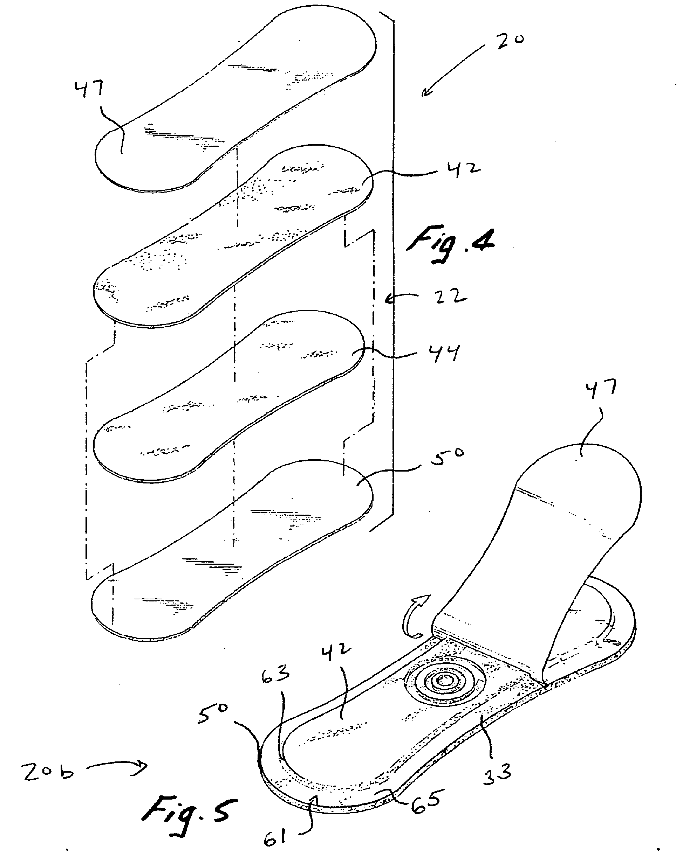

As depicted in FIG. 4, the main body 22 is of a laminate construction and preferably comprises a fluid-permeable cover layer 42, an absorbent system 44 and a fluid-impervious barrier layer 50. The absorbent system 44 may comprise a single layer of material or may comprise multiple layers. For example, the absorbent system may comprise a single layer core or it may include a transfer layer and a core.

In the embodiment of the invention shown in Figs. 1-3, an adhesive 33 for securing the napkin 20 to the body of a user is applied to the body facing surface 45 of the cover 42.

The napkin 20 further includes a tactile placement aid generally identified by the numeral 60. In the particular embodiment of the invention shown in Figs. 1-4, the placement aid 60 includes a plurality of concentric circles 62 arranged about a central circular land area 66. The concentric circles 62 and land area 66 may be formed by embossing the article with a pair of embossing rolls having a pattern corresponding in shape to the concentric circles 62 and central circular land area 66. Other techniques for forming the tactile placement aid 60 will also be apparent to those skilled in the art.

As best seen in Figs. 1 and 3, the concentric circles 62 are formed during the embossing process such that the concentric circles 62 and the central circular land area 66 extend outward relative to the non-embossed planar outer portions 64 of the barrier 50. In this manner, each concentric circle 62 and the central circular land area 66 is tactilely distinguishable to a user from the non-embossed portions 64 of the barrier 50. Each of the concentric circles 62 and the central circular land area 66 preferably extend out a distance "d" relative to the non-embossed planer portions 64 of the barrier 50. Preferably the distance "d" is at least .25 mm, more preferably at least .50 mm, and most preferably at least 1.0 mm. In this manner the user can tactilely distinguish the tactile placement aid 60 from the non-embossed regions of the article. In a preferred embodiment of the invention, the concentric circles 62 and central circular land area 66 are formed such that the geometric center of the central circular land area 66, and thus the geometric center of the tactile placement aid 60, is arranged at a location on the napkin 20 intended to be placed over the vaginal opening. In other words, the placement aid 60 is structured and arranged such that the geometric center thereof is located at a location on the napkin 20 intended to be aligned with the vaginal opening.

The adhesive 33 used in the article according to the present invention is preferably an adhesive based upon block copolymers, preferably, those which may include linear or radial co-polymer structures having the formula (A-B)ₓ wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or most preferably hydrogenated elastomers such as ethylene-butylene or ethylene-propylene or polyisobutylene, or combinations thereof, specifically, adhesives consisting of styrene-ethylene-butylene-styrene (SEBS) block copolymer and mineral oils, paraffinic or napthenic process oils, and optionally a suitable tackifying resins include natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic/aromatic petroleum resins and the hydrogenated derivatives thereof, and combinations thereof

The adhesive 33 may by of the type described in US Patent No. 6,191,189 to Cinelli et al. In particular, the adhesive may comprise:
from 0.5 to 20%, preferably 5% to 15%, by weight of a macromolecular polymeric substance or a mixture of such substances soluble or swellable in the below mentioned plasticiser(s). As not limiting examples such macromolecular or polymeric substances can be natural and/or synthetic such as natural gums or derivatives such as natural gums and gelatins, their derivatives and alginates; polyacrylics; polyvinyl alcohol; polyethylene oxide; polyvinylpyrrolidone (PVP) or polyvinylethers, their copolymers and derivatives; cellulose derivatives; Block Copolymer Thermoplastic Elastomers and preferably Styrenic Block Copolymers and more preferably the hydrogenated grades Styrol/Ethylene-Butylene/Styrol (SEBS), Styrene/Isoprene/Styrene (SIS), and Styrol/Ethylene-Propylene/Styrol (SEPS);
from 45 to 99.5% by weight, preferably from 51 to 99.5% by weight, of a plasticising substance or a mixture of plasticising substances, which are liquid at room temperature. As non-limiting examples the plasticiser can be water, various alcohols (like in particular glycerol), glycols and their ethers, polyglycols, liquid polybutenes, esters such phthalates, adipates, stearates, palmitates, sebacates, or myristates, natural or synthetic oils such as vegetable oils, mineral oils, or combinations thereof;
from 0% to 50% by weight of the composition, preferably from 0 to 600% by weight of the macromolecular polymeric substance of a tackifying resin whose main scope is to tailor the Tg especially in systems based on synthetic polymers;
from 0 to 10% and more preferably form 0 to 5% by weight of substances for facilitating and stabilising the gel and the gel forming process both of hydrophilic or hydrophobic liquid plasticisers. These may be for oily systems, e.g. the fatty acids of C₈ to C₂₂, their metallic salts and their polyoxo-derivatives; lanolin derivatives; silica; bentonite, montmorillonite and their derivatives; polyamides, waxes or mixtures thereof.

The adhesive may also be of the type described in US Patent No. 6,213,993 to Zacharias et al.. In particular the adhesive may comprise:
a rubber-based adhesive such as styrenebutadiene, polyisobutylene, polybutadiene and polyisoprene; a water soluble adhesive such as polyvinyl alcohol, polyvinyl acetate, and methyl cellulose; a hot melt adhesive such as block copolymers of styrene-butadienestyrene, styrene-isoprene-styrene, styrene-ethylenepropylene-styrene, styrene-ethylenebutylene-styrene and tetrablock copolymers such as styrene-ethylenepropylene-styrene-ethylenepropylene. Incorporated with the adhesives can be suitable tackifying resins and, if appropriate, oils.

Other adhesive types here include anhydrous gels consisting of 2-hydroxyethyl methacrylate polymer, polyethylene glycol and optionally water as taught in U.S. Patent No. 4,303,066 and polyurethane gels, as disclosed in U.S. Patent No. 4,661,099 , or silicone gels including commercial products such as Silgel 612 from Wacker Silicones (Adrian, MI) or SSA-9700 Soft Skin Adhesives Dow-Corning (Midland, MI).

The adhesive 33 is covered prior to use by a removable release member 47. The release member 47 may comprise a single layer structure or may comprise a laminate structure. For example, the release member may consist of a POLY SLIK^{®} brand paper, available from Loparex Inc., Willowbrook, IL. The internal surface of the release member 47 is provided with a release coating 53 to facilitate the removal of the release member 47 prior to use. The release member 47 is arranged such that prior to the removal of the release member 47, the coating 53 is in abutting face to face relationship with the adhesive 33. The release coating may 53 be a material based on polydimethylsiloxane chemistries, generically referred to as "silicones".

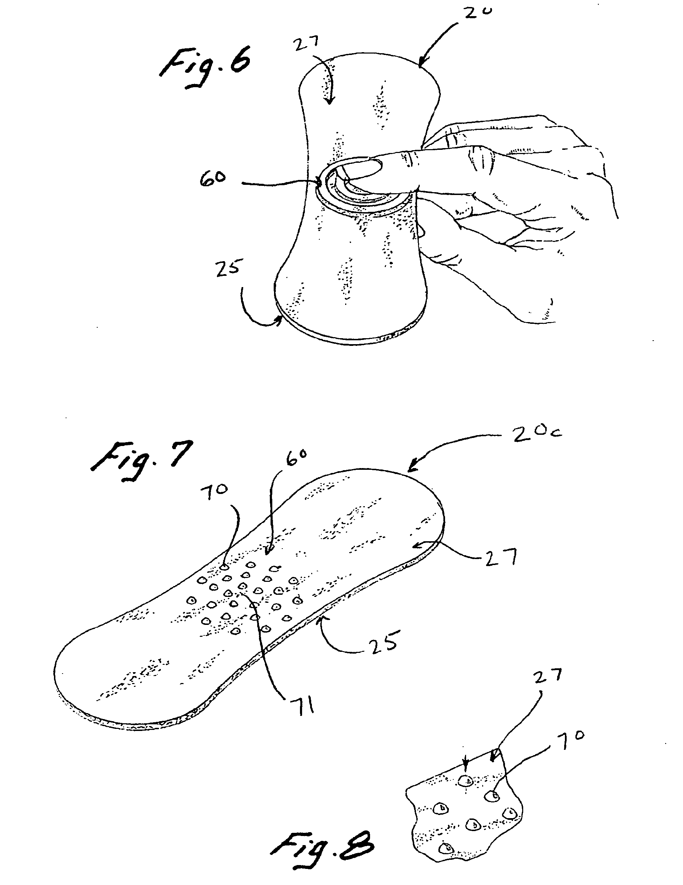

The method of applying the napkin 20 of the present invention to the body will now be described with reference to Figs. 1, 2 and 6. Prior to adhering the napkin 20 to the body, the user first removes from the body facing surface 45 of the cover 42 the removable release member 47, as shown in Figs. 1 and 2. Then, as shown in Fig. 6, the user tactilely locates the geometric center of the placement aid 60 and tactilely aligns the center of the placement aid 60 with the vaginal opening (not shown). After the placement aid 60 is aligned with the vaginal opening, the user then applies pressure to the garment facing surface 27 of the article so that the body facing surface 25 of the napkin 20 is pressed firmly against the body to thereby adhere the napkin 20 to the body. By using the above described method, the user can insure that the napkin 20 is arranged in the proper location without having to visually align the product.

A second embodiment of the present invention, a sanitary napkin 20b, is shown in Fig. 5. In the sanitary napkin 20b the barrier layer 50 is dimensioned so a portion 61 thereof extends outward relative to a terminal edge 63 of the cover 42. The adhesive 33 for attaching the article to the body is applied to a body facing surface 65 of the barrier portion 61. The napkin 20b is provided with a removable release member 47 to cover the adhesive 33 prior to use. As shown in Fig. 5 the release member 47 may be shaped such that it extends over the entire top surface of the cover 42 and barrier portion 61. Alternatively, the removable release member 47 may have a substantially oval shape (not shown) such that the release member 47 corresponds in shape to the barrier portion 61 and has a central open area (i.e. a central oval shaped through hole) that corresponds to the shape of the cover 42. The remaining structure of the embodiment of the invention 20b shown in Fig. 5 is the same as the embodiment described above with respect to Figs. 1-4.

A third embodiment of the present invention, a sanitary napkin 20c, is shown in Figs. 7 and 8. In the sanitary napkin 20c the tactile placement aid 60 is formed by a plurality of raised elements or protuberances 70. A detailed view of the raised elements 70 is provided in Fig. 8. The raised elements 70 may be formed, for example, from a non-contact adhesive applied to the garment facing surface 27 of the article

The raised elements 70 may be applied onto the substrate by any means known in the art, such as control coating, control fiberization, pattern coating, gravure coating, rotary screen printing, and spray coating. Equipment for coating the substrates is commercially available. One example is the DYNAFIBER, available through Nordson Company. Another example is the ITW, available through Omega Company. When applying raised elements 70 through a melt process, the time it takes to cool the applied coating affects the height of the raised elements. If the coating is not cooled quickly enough, the coating may penetrate the substrate to the extent that no raised element 70 is formed. To overcome this problem, an air knife that utilizes air, which may be chilled, may be utilized to quickly cool the applied coating and prevent tailing. The angle of contact between the air and the applied coating may also affect the height of the raised elements 70. The air typically contacts the coating at an angle of from about 10 to about 80°. The raised elements 70 may be of any shape including, but not limited to, lines, waves, interconnected patterns, circular dots, hexagons, hearts, diamonds, rectangles, stars, triangles and the like. The density, height, and diameter of the raised elements may vary so long as the elements can be easily tactilely perceived by the user.Preferably the raised elements 70 have a height of at least .25 mm, more preferably at least .50 mm, and most preferably at least 1.0 mm

The raised elements 70 are made of any suitable material that is easily tactilely perceptible to the user. Suitable materials include, but are not limited to, hot melt coatings, natural rubber, synthetic rubber, polyolefins, such as polyethylene and polypropylene, ethylene vinyl acetate, and thermoplastic elastomers. Colorants or pigments may be combined with the coating materials.

Suitable hot melt coatings for generating raised elements include HL-7471 W from H. B. Fuller Co., St. Paul, Minn., and REXTAC amorphous polyolefins, available through Huntsman Chemical. For example, hot melt coatings containing from about 15% to about 100% olefin polymer or a block copolymer, from about 0% to about 60% tackifying resin, and from about 0% to about 50% wax may be useful. Suitable olefin polymers include polymers:
a) wherein the olefin polymer is a homopolymer of ethylene, propylene, n-butene, butylene or isobutylene, with a melt flow index from 0.5 to 2500, such as Ateva^{®} polymers from AT plastics; Escorene^{®} and Vistanex^{®} polymers from Exxon Chemical, Duraflex^{®} polymers from Shell Chemical, Epolene^{®} polymers from Eastman Chemical, and Vestoplast^{®} polymers from Creanova;
b) wherein the olefin polymer is a copolymer of ethylene and a co-monomer, such as vinyl acetate, acrylic acid, methacrylic acid, ethyl acrylate, methyl acrylate, n-butyl acrylate vinyl silane or maleic anhydride, such as Ateva^{®} polymers from AT plastics, Elvax^{®} polymers from DuPont, Escorene^{®} and Optema^{®} polymers from Exxon Chemical, and Primacor^{®} polymers from Dow Chemical; and
c) wherein the olefin polymer is a terpolymer of ethylene and co-monomers, such as vinyl acetate, acrylic acid, methacrylic acid, ethyl acrylate, methyl acrylate, n-butyl acrylate vinyl silane or maleic anhydride, such as Ateva^{®} polymers from AT plastics, Nucrel^{®} polymers from DuPont, and Escor^{®} polymers from Exxon Chemical.
Suitable block copolymers include block copolymers having a linear or a radial structure such that the structure (A--B)ₓ where A is consists essentially of a polyvinylarene block, and B consists essentially of poly(monoalkenyl) block, and x denotes the number of polymeric arms, where x is greater than or equal to one are also useful. Block B may be selected from conjugated diene elastomers such as polybutadiene or polyisoprene and hydrogenated elastomers such as ethylene-butylene or ethylene-propylene. Suitable examples of these types of polymers include Kraton^{®} elastomers from Shell Chemical Company, Vector^{®} elastomers from Dexco, Solprene^{®} elastomers from Enichem Elastomers and Stereon^{®} from elastomers Firestone Tire & Rubber Co. When the hot melt coatings contain block copolymers, it is preferable for the coating to contain from about 15% to about 50% block copolymer.

Suitable tackifying resins include any compatible resin or mixture thereof selected from the group consisting of a) natural and modified rosins; b) glycerol and pentaerythritol esters of natural and modified rosins; c) polyterpene resins; d) copolymers and terpolymers of natural terpenes; e) phenolic modified terpene resins and the hydrogenated derivatives thereof, f) aliphatic petroleum resins and the hydrogenated derivatives thereof; g) aromatic petroleum resin and the hydrogenated derivatives thereof; and h) aliphatic/aromatic petroleum resins and the hydrogenated derivatives thereof, such as Foral^{®} resin, Staybelite^{®} resin, Poly-pale^{®} resin, Permalyn^{®} resin, Pentalyn^{®} resin, Adtac^{®} resin, Piccopale^{®} resin, Piccotac^{®} resin, Hercotac^{®} resin, Regalrez^{®} resin, and Piccolyte^{®} resin from Hercules, Escorez^{®} resin from Exxon Chemical, Wingtack^{®} resin from Goodyear Tire & Rubber Co., Arkon^{®} resin from Arakawa Chemicals, Zonatac^{®} resin, Zonarez^{®} resin and Zonester^{®} resin from Arizona Chemical and Nevtac^{®} resin from Neville Chemical Company.

Suitable waxes include, but are not limited to, paraffins, Fischer-tropsh, and microcrystalline waxes, and combinations thereof. Suitable microcrystalline waxes include, but are not limited to, BE SQUARE 175 microwax, available from Bareco Division, Petrolite Corporation, and M-5165 from Moore & Munger, Shelton, Conn. Suitable polyethylene waxes include, but are not limited to, H- 101 from Exxon Chemical, Houston, Tex. Suitable Fischer-Tropsch waxes include, but are not limited to, Paraflint Wax from Schumann Sasol, Hamburg, Germany.

As shown in Fig.7, the protuberances 70 collectively define a geometric shape, in the case of the specific example shown in Fig. 7, a circle. Preferably the protuberances 70 are arranged such that the geometric center 71 of the shape defined by the protuberances 70 is located at a location on the article adapted to be aligned with a vaginal opening of a user. The remaining structure of the embodiment of the invention 20c shown in Figs. 7-8 is the same as the embodiment described above with respect to Figs. 1-4.

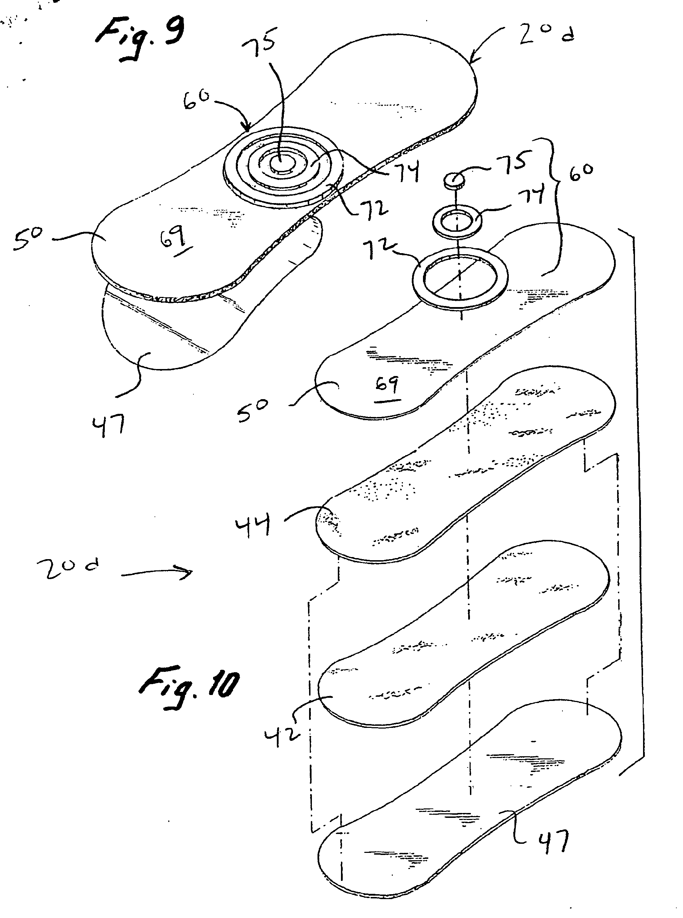

Still another embodiment of the present invention, a sanitary napkin 20d, is shown in Figs. 9-10. In the sanitary napkin 20d the tactile placement aid 60 is formed from at least one additional layer of material applied to the outer surface 69 of the barrier 50. In the specific embodiment shown in Figs.9-10 the tactile placement aid 60 comprises a first ring 72 of material, a second concentric ring 74 of material, and a central circular land area 75. The material used to form the first ring 72, second ring 74, and the central circular land area 75 may be a non-woven material, film material or other materials used in sanitary protection articles. The material first ring 72, second ring 74, and the central circular land area 75 may, for example, be applied to the outer surface 69 of the barrier using an adhesive or other known methods. The material used to form the tactile placement aid 60 preferably has a thickness of at least .25 mm, more preferably at least .50 mm, and most preferably at least 1.0 mm. In a preferred embodiment of the invention, the concentric rings 72 and 74 and central circular land area 75 are formed such that the geometric center of the central circular land area 75, and thus the geometric center of the tactile placement aid 60, is arranged at a location on the napkin 20d intended to be placed over the vaginal opening. In other words, the placement aid 60 is structured and arranged such that the geometric center thereof is located at a location on the napkin 20d intended to be aligned with the vaginal opening.

Although various embodiments of the tactile placement aid 60 have been described above other embodiments are possible as long as the tactile placement aid is tactilely perceptible to a user from a garment facing side of the article.

### Main Body--Cover Layer

The cover layer 42 may be a relatively low density, bulky, high-loft non-woven web material. The cover layer 42 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 42 has a basis weight in the range of about 10 gsm to about 75 gsm.

Bi-component fibers may be made up of a polyester layer and a an polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer.

The cover layer 42 preferably has a relatively high degree of wettability, although the individual fibers comprising the cover may not be particularly hydrophilic. The cover material should also contain a great number of relatively large pores. This is because the cover layer 42 is intended to take-up body fluid rapidly and transport it away from the body and the point of deposition. Therefore, the cover layer contributes little to the time taken for the napkin to absorb a given quantity of liquid (penetration time).

Advantageously, the fibers which make up the cover layer 42 should not lose their physical properties when they are wetted, in other words they should not collapse or lose their resiliency when subjected to water or body fluid. The cover layer 42 may be treated to allow fluid to pass through it readily. The cover layer 42 also functions to transfer the fluid quickly to the other layers of the absorbent system 44. Thus, the cover layer 42 is advantageously wettable, hydrophilic and porous. When composed of synthetic hydrophobic fibers such as polyester or bi-component fibers, the cover layer 42 may be treated with a surfactant to impart the desired degree of wettability.

In one preferred embodiment of the present invention the cover is made from a spunlace nonwoven material having from about 0 to about 100% polyester and from about 0 to about 100% rayon. The spunlace material may also be made from about 10% to about 65% rayon and from about 35% to about 90% polyester. In lieu of, and/or in combination with the polyester, polyethylene, polypropylene or cellulosic fiber may be used with the rayon. Optionally, the material used for the cover layer may include binders such as thermoplastic binders and latex binders.

Alternatively, the cover layer 42 can also be made of polymer film having large pores. Because of such high porosity, the film accomplishes the function of quickly transferring body fluid to the inner layers of the absorbent system. Apertured co-extruded films such described in U.S. Pat. No. 4,690,679 and available on sanitary napkins sold by Johnson & Johnson Inc. of Montreal, Canada could be useful as cover layers in the present invention.

The cover layer 42 may be embossed to the remainder of the absorbent system 44 in order to aid in promoting hydrophilicity by fusing the cover to the next layer. Such fusion may be effected locally, at a plurality of sites or over the entire contact surface of cover layer 42 and absorbent system 44. Alternatively, the cover layer 42 may be attached to the absorbent system 44 by other means such as by adhesion.

### Main Body -- Absorbent System

The absorbent system 44 may comprise a single layer of material or may comprise multiple layers. In one embodiment, the absorbent system 44 is a blend or mixture of cellulosic fibers and superabsorbent disposed in and amongst fibers of that pulp.

Cellulosic fibers that can be used in the absorbent system 44 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The absorbent system 44 can contain any superabsorbent polymer (SAP), which SAPs are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc..

### Main Body-Barrier Layer

Underlying the absorbent layer 44 is a barrier layer 50 comprising liquid-impervious film material so as to prevent liquid that is entrapped in the absorbent system 44 from egressing the sanitary napkin and staining the wearer's undergarment. The barrier layer 50 is preferably made of polymeric film, although it may be made of liquid impervious, air-permeable material such as repellent-treated non-woven or micropore films or foams.

The barrier layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 42 and the barrier layer 50 are joined along their marginal portions so as to form an enclosure or flange seal that maintains the absorbent layer 44 captive.

Any or all of the cover, absorbent layer, transfer layer, backsheet layer, and adhesive layers may be colored. Such coloring includes, but is not limited to, white, black, red, yellow, blue, orange, green, violet, and mixtures thereof. Color may be imparted according to the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like. Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

The absorbent article may include other known materials, layers, and additives, such as, foam, net-like material, perfumes, medicaments or pharmaceutical agents, moisturizers, odor control agents, and the like. The absorbent article can optionally be embossed with decorative designs.

The absorbent article may be packaged as unwrapped absorbent articles within a carton, box or bag. The consumer withdraws the ready-to-use article as needed. The absorbent article may also be individually packaged (each absorbent article encased within an overwrap).

Also contemplated by the present invention are asymmetrical and symmetrical absorbent articles having parallel longitudinal edges, dog bone- or peanut-shaped, as well as articles having a tapered construction for use with thong-style undergarments.

From the foregoing description, one skilled in the art can ascertain the essential characteristics of this invention, and without departing from the scope thereof, can make various changes and modifications. Embodiments set forth by way of illustration are not intended as limitations on the variations possible in practicing the present invention.

## Claims

1. A body-attachable absorbent article comprising:
a body facing surface;
a garment facing surface;
an adhesive arranged on said body facing surface for securing said article to the body; and
a tactile placement aid perceptible to a user on a non-visual basis and that is tactilely perceptible to a user from said garment facing surface of said article
wherein said tactile placement aid comprises a first tactilely perceptible element and a least a second tactilely perceptible element arranged within said first tactilely perceptible element.

2. The body-attachable absorbent article according to claim 1, wherein said tactile placement aid is arranged at a location on said absorbent article adapted to be aligned with a vaginal opening of a user.

3. The body-attachable absorbent article according to any previous claim, wherein said tactile placement aid has a geometric center, said geometric center being arranged at a location on said absorbent article adapted to be aligned with a vaginal opening of a user.

4. The body-attachable absorbent article according to any previous claim, wherein said tactile placement aid comprises at least one tactile circle that is tactilely perceptible to a user from said garment facing surface of said article.

5. The body-attachable absorbent article according to claim 4, wherein said tactile placement aid comprises a plurality of concentric circles.

6. The body-attachable absorbent article according to any previous claim, wherein said tactilely perceptible element extends outwardly relative to said garment facing surface at least .25 mm.

7. The body-attachable absorbent article according to claim 6, wherein said tactilely perceptible element extends outwardly relative to said garment facing surface at least .50 mm.

8. The body-attachable absorbent article according to claim 7, wherein said tactilely perceptible element extends outwardly relative to said garment facing surface at least 1.0 mm.

9. The body-attachable absorbent article according to any previous claim, wherein said article is one of a sanitary napkin, liner and adult-incontinence product.

10. The body-attachable absorbent article according to any previous claim, wherein said tactile placement aid is formed by embossing said article.

11. The body-attachable absorbent article according to any previous claim, wherein said tactile placement aid comprises at least one protuberance extending from said garment facing surface.

12. The body-attachable absorbent article according to claim 11, wherein said tactile placement aid comprises a plurality of protuberances.

13. The body-attachable absorbent article according to claim 12, wherein said plurality of protuberances define a geometric shape.

14. The body-attachable absorbent article according to claim 13, wherein said geometric shape has a center, said protuberances being arranged on said garment facing surface such that said center is located at a portion of the article adapted to be arranged over a vaginal opening of a user.

15. The body-attachable absorbent article according to any previous claim, wherein said tactile placement aid comprises at least one material layer applied to said garment facing surface.

16. A body-attachable absorbent article according to any previous claim 1-15, further comprising:
a cover layer;
a barrier layer,
and wherein said adhesive is applied to a body facing side of said cover layer for securing said article to the body.

17. The body-attachable absorbent article according to claim 16, further comprising:
an absorbent system arranged between said cover layer and said barrier layer.

18. A body-attachable absorbent article according to any previous claim 1-15, wherein said barrier layer is structured and arranged so that a portion thereof extends beyond a terminal edge of said cover layer; and wherein
said adhesive for securing said article to the body is applied to a body facing side of said portion of said barrier layer that extends beyond said terminal edge of said cover.

19. A method of applying a body-attachable absorbent article, according to any previous claim 1-18, to the body, comprising:
tactilely locating the tactile placement aid on a body-attachable absorbent article;
tactilely aligning the tactile placement aid with a vaginal opening of a user; and
adhering the body-attachable absorbent article against a body of the user so that said tactile placement aid is aligned with said vaginal opening of said user.

## Patentansprüche

1. Am Körper anbringbarer absorbierender Artikel, umfassend:
eine dem Körper zugewandte Oberfläche;
eine dem Kleidungsstück zugewandte Oberfläche;
ein Haftmittel, das auf der dem Körper zugewandten Oberfläche aufgebracht ist, um den Artikel am Körper zu befestigen; und
eine kühlbare Platzierungshilfe, die für einen Anwender nicht visuell wahrnehmbar ist und für eine Benutzerin durch Fühlen von der dem Kleidungsstück zugewandten Oberfläche des Artikels wahrnehmbar ist,
wobei besagte fühlbare Platzierungshilfe ein erstes durch Fühlen wahrnehmbares Element und wenigstens ein zweites durch Fühlen wahrnehmbares Element umfasst, das im ersten durch Fühlen wahrnehmbaren Element angeordnet ist.

2. Am Körper anbringbarer absorbierender Artikel nach Anspruch 1, **dadurch gekennzeichnet, dass** die fühlbare Platzierungshilfe an einer Stelle auf dem absorbierenden Artikel angeordnet ist, um mit einer Vaginalöffnung einer Benutzerin ausgerichtet zu werden.

3. Am Körper anbringbar absorbierender Artikel nach einem der vorstehenden Ansprüche, **dadurch gekennzeichnet, dass** die fühlbare Platzierungshilfe ein geometrisches Zentrum aufweist, wobei das geometrische Zentrum an einer Stelle auf dem absorbierenden Artikel anordbar ist, um mit einer Vaginalöffnung einer Benutzerin ausgerichtet zu werden.

4. Am Körper anbringbarer absorbierender Artikel nach einem der vorstehenden Ansprüche, **dadurch gekennzeichnet, dass** die fühlbare Platzierungshilfe wenigstens einen fühlbaren Kreis aufweist, der für eine Benutzerin durch Fühlen von der dem Kleidungsstück zugewandten Oberfläche des Artikels wahrnehmbar ist.

5. Am Körper anbringbarer absorbierender Artikel nach Anspruch 4, **dadurch gekennzeichnet, dass** die fühlbare Platzierungshilfe eine Mehrzahl von konzentrischen Kreisen umfasst.

6. Am Körper anbringbarer absorbierender Artikel nach einem der vorstehenden Ansprüche, **dadurch gekennzeichnet, dass** sich das durch Fühlen wahrnehmbare Element relativ zu der dem Kleidungsstück zugewandten Oberfläche um wenigstens 0,25 mm nach außer erstreckt.

7. Am Körper anbringbarer absorbierender Artikel nach Anspruch 6, **dadurch gekennzeichnet, dass** sich das durch Fühlen wahrnehmbare Element relativ zu der dem Kleidungsstück zugewandten Oberfläche um wenigstens 0,50 mm nach außen erstreckt.

8. Am Körper anbringbarer absorbierender Artikel nach Anspruch 7, **dadurch gekennzeichnet, dass** sich das durch Fühlen wahrnehmbare Element relativ zu der dem Kleidungsstück zugewandten Oberfläche um wenigstens 1,0 mm nach außen erstreckt.

9. Am Körper anbringbarer absorbierender Artikel nach einem der vorstehenden Ansprüche, **dadurch gekennzeichnet, dass** der Artikel eine Damenbinde, eine Slipeinlage oder ein Inkontinenzprodukt für Erwachsene ist.

10. Am Körper anbringbarer absorbierender Artikel nach einem der vorstehenden Ansprüche, **dadurch gekennzeichnet, dass** die fühlbare Platziemngshilfe durch Prägen des Artikels geformt ist.

11. Am Körper anbringbarer absorbierender Artikel nach einem der vorstehenden Ansprüche, **dadurch gekennzeichnet, dass** die fühlbare Platzierungshilfe wenigstens eine Erhebung aufweist, die sich von der dem Kleidungsstück zugewandten Oberfläche weg erstreckt.

12. Am Körper anbringbarer absorbierender Artikel nach Anspruch 11, **dadurch gekennzeichnet, dass** die ertastbare Platzierungshilfe eine Mehrzahl von Erhebungen aufweist.

13. Am Körper anbringbarer absorbierender Artikel nach Anspruch 12, **dadurch gekennzeichnet, dass** die Mehrzahl der Erhebungen eine geometrische Form definieren.

14. Art Körper anbringbarer absorbierender Artikel nach Anspruch 13, **dadurch gekennzeichnet, dass** die geometrische Form ein Zentrum aufweist, wobei die Erhebungen auf der dem Kleidungsstück zugewandten Oberfläche so angeordnet sind, dass sich das Zentrum auf einem Abschnitt des Artikels befindet, um über einer Vaginalöffnung der Benutzerin ausgerichtet zu werden.

15. Am Körper anbringbarer absorbierender Artikel nach einem der vorstehenden Ansprüche, **dadurch gekennzeichnet, dass** die fühlbare Platzierungshilfe wenigstens eine Materialschicht aufweist, die auf der dem Kleidungsstück zugewandten Oberfläche aufgebracht ist.

16. Am Körper anbringbarer absorbierender Artikel nach einem der vorstehenden Ansprüche 1-15, ferner umfassend:
eine Deckschicht;
eine Sperrschicht,
wobei das Haftmittel auf der dem Körper zugewandten Oberfläche der Deckschicht aufgebracht ist, um den Artikel am Körper zu befestigen.

17. Am Körper anbringbarer absorbierender Artikel nach Anspruch 1, ferner umfassend:
ein absorbierendes System, das zwischen der Deckschicht und der Sperrschicht angeordnet ist.

18. Am. Körper anbringbarer absorbierender Artikel nach einem der vorstehenden Ansprüche 1-15, **dadurch gekennzeichnet, dass** die Sperrschicht so strukturiert und angeordnet ist, dass ein Teil von ihr sich über eine Abschlusskante der Deckschicht hinaus erstreckt; und
das Haftmittel zum Befestigen des Artikels am Körper auf der dem Körper zugewandten Seite des Abschnitts der Sperrschicht aufgebracht ist, der sich über die Abschlusskante der Deckschicht hinaus erstreckt.

19. Verfahren zum Anbringen eines am Körper anbringbaren absorbierenden Artikels nach eirem der vorangehenden Ansprüche 1-18 am Körper, umfassend:
Orten der fühlbaren Platzierungshilfe auf dem am Körper anbringbaren absorbierenden Artikel durch Fühlen;
Ausrichten der fühlbaren Platzierungshilfe mit einer Vaginalöffnung einer Benutzerin durch Fühlen; und
Kleben des am Körper anbringbaren absorbierenden Artikels an dem Körper der Benutzerin derart, dass die fülhlbare Platzierungshilfe mit der Vaginalöffnung der Benutzerin ausgerichtet ist.

## Revendications

1. Article absorbant pouvant être fixé au corps comprenant :
une surface orientée vers le corps ;
une surface orientée vers le vêtement ;
un adhésif disposé sur ladite surface orientée vers le corps pour fixer ledit article au corps ; et
un assistant de placement tactile perceptible pour un utilisateur sur une base non visuelle et qui est perceptible d'un point de vue tactile par l'utilisateur sur la surface orientée vers le vêtement dudit article,
dans lequel ledit assistant de placement tactile comprend un premier élément perceptible d'un point de vue tactile et au moins un second élément perceptible d'un point de vue tactile, disposé dans ledit premier élément perceptible d'un point de vue tactile.

2. Article absorbant pouvant être fixé au corps selon la revendication 1, dans lequel ledit assistant de placement tactile est disposé à un endroit sur ledit article absorbant permettant l'alignement avec une ouverture vaginale d'un utilisateur.

3. Article absorbant pouvant être fixé au corps selon l'une quelconque des revendications précédentes, dans lequel ledit assistant de placement tactile a un centre géométrique, ledit centre géométrique étant disposé à un endroit sur ledit article absorbant permettant l'alignement avec une ouverture vaginale d'un utilisateur.

4. Article absorbant pouvant être fixé au corps selon l'une quelconque des revendications précédentes, dans lequel ledit assistant de placement tactile comprend au moins un cercle tactile qui est perceptible d'un point de vue tactile pour un utilisateur partir de ladite surface orientée vers le vêtement dudit article.

5. Article absorbant pouvant être fixé au corps selon la revendication 4, dans lequel ledit assistant de placement tactile comprend une pluralité de cercles concentriques.

6. Article absorbant pouvant être fixé au corps selon l'une quelconque des revendications précédentes, dans lequel ledit élément perceptible d'un point de vue tactile s'étend vers l'extérieur relativement à ladite surface orientée vers le vêtement sur au moins 0,25 mm.

7. Article absorbant pouvant être fixé au corps selon la revendication 6, dans lequel ledit élément perceptible d'un point de vue tactile s'étend vers l'extérieur relativement à ladite surface orientée vers le vêtement sur au moins 0,50 mm.

8. Article absorbant pouvant être fixé au corps selon la revendication 7, dans lequel ledit élément perceptible d'un point de vue tactile s'étend vers l'extérieur relativement à ladite surface orientée vers le vêtement sur au moins 1,0 mm.

9. Article absorbant pouvant être fixé au corps selon l'une quelconque des revendications précédentes, dans lequel ledit article est une serviette hygiénique, une doublure et un produit d'incontinence pour adulte.

10. Article absorbant pouvant être fixé au corps selon l'une quelconque des revendications précédentes, dans lequel ledit assistant de placement tactile est formé en estampant ledit article.

11. Article absorbant pouvant être fixé au corps selon l'une quelconque des revendications précédentes, dans lequel ledit assistant de placement tactile comprend au moins une protubérance s'étendant depuis ladite surface orientée vers le vêtement.

12. Article absorbant pouvant être fixé au corps selon la revendication 11, dans lequel ledit assistant de placement de tactile comprend une pluralité de protubérances.

13. Article absorbant pouvant être fixé au corps selon la revendication 12, dans lequel ladite pluralité de protubérances définit une forme géométrique.

14. Article absorbant pouvant être fixé au corps selon la revendication 13, dans lequel ladite forme géométrique a un centre, lesdites protubérances étant disposées sur ladite surface orientée vers le vêtement, de sorte que ledit centre soit situé sur une portion de l'article adaptée pour être disposée au niveau d'une ouverture vaginale d'un utilisateur.

15. Article absorbant pouvant être fixé au corps selon l'une quelconque des revendications précédentes, dans lequel ledit assistant de placement tactile comprend au moins une couche de matériau appliquée à ladite surface orientée vers le vêtement.

16. Article absorbant pouvant être fixé au corps selon l'une quelconque des revendications 1-15, comprenant en outre :
une couche de couverture,
une couche barrière
et dans lequel ledit adhésif est applique un côté orientée vers le corps de ladite couche de couverture pour fixer ledit article au corps.

17. Article absorbant pouvant être fixé au corps selon la revendication 16, comprenant en outre un système absorbant disposé entre ladite couche de couverture et ladite couche barrière.

18. Article absorbant pouvant être fixé au corps selon l'une quelconque des revendications 1-15, dans lequel ladite couche barrière est structurée et disposée de sorte qu'une portion de celle-ci s'étende au-delà d'un bord d'extrémité de ladite couche de couverture ; et dans lequel
ledit adhésif pour fixer ledit article au corps est appliqué à un côté orienté vers le corps de ladite portion de ladite couche barrière qui s'étend au-delà dudit bord d'extrémité de ladite couverture.

19. Procédé d'application d'un article absorbant pouvant être fixé au corps, selon l'une quelconque des revendications précédentes 1-18, sur le corps, comprenant :
le placement tactile de l'assistant au placement tactile sur un article absorbant pouvant être fixé au corps ;
l'alignement tactile de l'assistant au placement tactile avec une ouverture vaginale d'un utilisateur ; et
l'adhésion de l'article absorbant pouvant être fixé au corps contre le corps d'un utilisateur, de sorte que ledit assistant de placement tactile soit aligné avec ladite ouverture vaginale dudit utilisateur.
